# EUROPEAN PATENT APPLICATION

(11) **EP 4 023 252 A1**
(43) Date of publication of application: **06.07.2022**
(21) Application number: 21215049.4
(22) Date of filing: 16.12.2021
(51) Int. Cl.: A61L 2/07, A47L 11/40, B08B 3/00, F22B 1/28

(54) **VAPOR DIFFUSER**

(30) Priority: 30.12.2020 IT 202000032804
(71) Applicant: Capitani S.r.l., 22043 Solbiate con Cagno (CO) (IT)
(72) Inventor: CAPITANI, Gionata, 22070 Albiolo (CO) (IT)
(74) Representative: Parisi, Luigi

(57) **Abstract**

A vapor diffuser (1) comprising a handle (2) adapted to be gripped by the hand of a user is described; said handle (2) comprising an inlet (3) for the vapor coming from a boiler, a heat exchanger (4) accommodated inside said handle (2), and an outlet for delivering high temperature vapor, characterized by comprising a delivering device adapted to be mounted at the outlet (5) comprising a bushing (6) and a grid (8) comprising a plurality of holes (7) for the passage of the vapor; said grid (8) and said bushing (6) being made of conductive material.

## Description

### Field of the invention

The present invention concerns the field of the vapor delivering apparatuses for the cleaning and sanitizing of environments. In particular, the present invention concerns a vapor diffuser adapted to be applied onto a machine for cleaning and sanitizing environments by means of vapor.

### Known art

As is known, apparatuses for delivering and dispensing vapor and used to carry out operations for cleaning and/or sterilizing paved surfaces and the like by delivering vapor in large quantities are present on the market. Such apparatuses essentially comprise a machine body integrating a boiler to which an appropriate vapor diffuser can be connected through a flexible pipe extending from the machine body.

Generally, the vapor diffusers comprise a handle adapted to be gripped by the hand of a user.

The handle comprises an inlet for the vapor coming from the boiler, a heat exchanger accommodated inside the handle and an outlet for delivering high temperature vapor, generally a delivery nozzle.

With the aid of such apparatuses, it is possible to achieve good results in terms of cleaning and sterilizing the surfaces treated.

The Applicant has however observed that although it is favorable to deliver vapor at a temperature above 150°C, such as to optimize the cleaning effectiveness, the vapor suffers a significant drop in temperature as soon as it is delivered, thus decreasing the sanitizing and disinfecting effectiveness.

The Applicant has thus faced with the problem of providing a diffuser which allows to deliver vapor at a temperature above 150°C and to ensure that the vapor delivered does not suffer an immediate temperature drop, also at a distance of a few milliliters from the delivery.

### Summary of the invention

Thus the invention, in a first aspect thereof, concerns a vapor diffuser comprising a handle adapted to be gripped by the hand of a user; said handle comprising an inlet for the vapor coming from a boiler, a heat exchanger accommodated inside said handle, and an outlet for delivering high temperature vapor, characterized by comprising a delivering device adapted to be mounted at the outlet comprising a bushing and a grid comprising a plurality of holes for the passage of the vapor; the grid and the bushing being made of conductive material.

Thanks to these characteristics, the Applicant has ascertained that the diffuser of the invention can delivery vapor at a temperature above 150°C, such as to optimize the cleaning effectiveness, while being able not to suffer a significant temperature drop as soon as it is delivered and/or a few millimeters from the point of delivery. Without wishing to bind himself to any particular theory, the Applicant believes that, in the bushing, the vapor is not free to be delivered but is obstructed by the grid.

This way, a turbulent flow of vapor is created inside the bushing, which raises the temperature of the vapor itself, part of the heat of the turbulent flow is transferred to the bushing itself and to the grid which, being made of conductive material, further raise their temperature and partially release it to the surrounding environment in the proximity of the delivery point.

In the aforesaid aspect, the present invention can have at least one of the preferred characteristics described hereunder.

Preferably, the cylindrical bushing and the grid are made of metal alloys.

Preferably, the bushing extends around an axis and has a substantially cylindrical shape for at least 50% of its extent; said bushing has a first end placed at said outlet and a second end away from said outlet.

Advantageously, the grid extends transversely to the axis at a distance of at least 10 mm from the outlet.

Preferably, the grid extends transversely to the axis at a maximum distance of 40 mm from the outlet.

Conveniently, the grid comprises a surface of at least 18 mm².

Preferably, the grid comprises a surface of up to 1500 mm².

Conveniently, the holes of the grid have a minimum vapor passage surface of 5000 mm.

Preferably, the holes of said grid have a diameter greater than or equal to 0.2 mm. Advantageously, the holes of said grid have a diameter less than or equal to 3 mm. Further characteristics and advantages of the invention will become clearer in the detailed description of some preferred, although not limiting, embodiments of a vapor diffuser according to the present invention.

### Brief description of the drawings

Such description will be explained hereunder with reference to the accompanying drawings, provided by way of example only and thus not limiting, in which:
- figure 1 shows a schematic perspective view of a machine for the cleaning and sanitizing of environments, provided with a vapor diffuser according to the present invention;
- figure 2 is a sectional schematic view of the vapor diffuser according to the present invention; and
- figure 3 is a front view of the grid of the vapor diffuser according to the present invention.

### Detailed description of embodiments of the invention

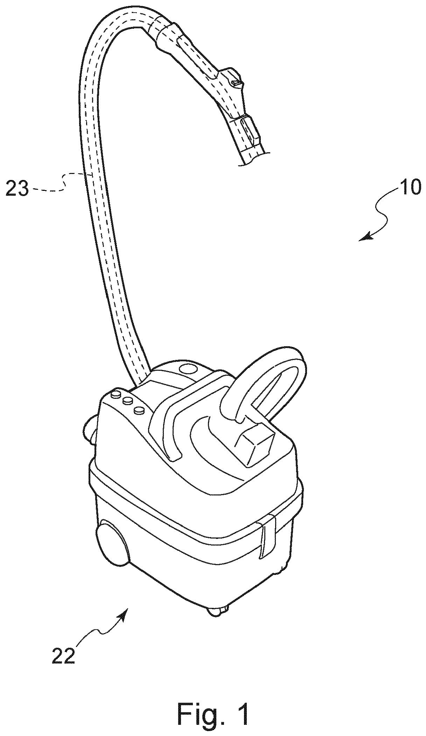

With reference to the figures, and in particular to figures 1 - 3, a cleaning apparatus for delivering and dispensing vapor according to the present invention is denoted by the numerical reference 10.

The cleaning apparatus 10 comprises a machine body 22 of a substantially box-shaped structure, inside which an electrically-operated boiler is housed for producing vapor.

Loading means are preferably combined with the boiler, which essentially comprise a loading pump which, for example operated by a float switch combined with the boiler, provides to draw water from a loading tank engaged above the machine body to send it into the boiler itself, such as the filling level of the latter is kept within predetermined limits.

An inflow channel which, as will become clearer hereunder, is used to connect the boiler itself with a delivery tube 23 extending outside the machine body 22, extends from the top of the boiler. Such delivery tube 23 converges into a vapor diffuser 1 that can be removably combined with the apparatus 10.

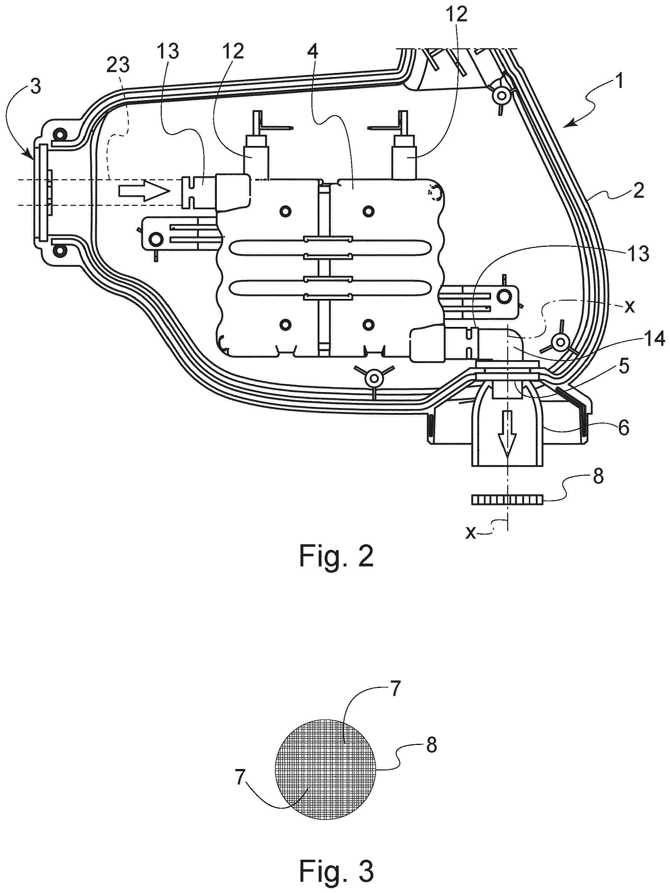

A vapor diffuser 1 according to the present invention is shown in section and partially exploded in figure 2.

The vapor diffuser 1 comprises a handle 2 adapted to be gripped by the hand of a user. The handle 2 has, at an end thereof, an inlet 3 for the vapor coming from the boiler through the duct, a heat exchanger 4 accommodated inside the handle 2 and an outlet 5 for delivering high temperature vapor.

The heat exchanger 4 has a box-shaped body 11, a duct for the passage of a liquid to be heated, made of steel and extending with a curvilinear pattern, preferably sinusoidal, inside the box-shaped body 11.

The box-shaped body 11, preferably parallelepiped-shaped, is made of aluminum.

The sinusoidal duct, not directly shown in figure 2, extends inside the box-shaped body 11 between an inlet and an outlet of the liquid/vapor to be heated, which are respectively placed at the inlet 4 and the outlet 5.

Along its course or path, the sinusoidal duct is in contact, in a known way, with a heating element, such as an electric coil 12 which, by transferring heat to the sinusoidal duct, allows to heat the water/vapor circulating therein.

The heat exchanger 4 further comprises two push-in devices 13 for coupling the sinusoidal duct respectively with a small inlet tube, not shown in the figure, and an elbow-like joint 14 which connects the heat exchanger 4 to the outlet 5.

The vapor diffuser 1 comprises a delivering element 15 adapted to be mounted at the outlet 5, preferably in fluidic connection with the elbow-like joint 14, such as to increase the temperature of the outflow vapor, up to more than 150°C, preferably more than 170°C. The delivering element 15 comprises a bushing 6 and a grid 8, which is arranged at an end of the bushing 6 and comprises a plurality of holes 7 for the passage of the vapor.

The grid 8 and the bushing 6 are made of conductive material.

Preferably, the grid 8 and the bushing 6 are made of a conductive metal alloy such as, once they have been heated by the vapor being delivered, they themselves contribute to maintaining and raising the temperature of the vapor being delivered.

Without intending to bind himself to any particular theory, the Applicant believes that the vapor, while crossing the bushing, is not free to be delivered but is obstructed by the grid. This way, a turbulent flow of vapor is created inside the bushing, which raises the temperature of the vapor itself, part of the heat of the turbulent flow is transferred to the bushing 6 itself and to the grid 8 which, being made of conductive material, further raise their temperature and partially release it to the surrounding environment in the proximity of the delivery point.

The bushing 6 extends around an axis X-X and has a substantially cylindrical shape for at least 50% of its extent.

Preferably, aside from a short connection area with an elbow-like joint 14, the bushing 6 has a substantially cylindrical shape along its whole extent.

The bushing 6 thus has a first end placed at the outlet 5 of the handle 2 and a second end away from the outlet 5 and bearing the grid 8.

In the embodiment shown in the figures, the grid 8 extends transversely to the axis X-X at a distance of at least 10 mm from the outlet 5.

Preferably, the grid extends transversely to the axis X-X at a maximum distance of 40 mm from the outlet 5.

In the embodiment shown in the figures, the grid 8 has a substantially circumferential shape.

The bushing 8 could have another shape without departing from the protection scope of the present invention.

The bushing 6 is internally hollow between the outlet 5 and the grid 8.

The bushing 6 has an inner section orthogonal to the axis X-X substantially coinciding with the surface of the grid 8.

The grid 8 comprises a surface of at least 18 mm².

Preferably, the grid 8 has a surface of up to 1500 mm².

Preferably, at least 30% of the plan surface of the grid 8 is covered with the through-holes 7.

In the embodiment shown in the figures, at least 70% of the plan surface of the grid 8 is covered with the through-holes 7.

In the embodiment shown in the figures, the holes are square-shaped but could however have any other shape without departing from the protection scope of the present invention. The through-holes 7 have a maximum size greater than or equal to 0.2 mm.

Preferably, the through-holes 7 of the grid have a maximum size less than or equal to 3 mm.

In the case shown in the figure, maximum size means one side by which the square has the largest size.

In the event of square-shaped holes, the maximum size is coincident with one side, in the event of circular-shaped holes 7 with the diameter, in the event of other polygonal shapes with the diameter of a circumference circumscribing the polygonal shape.

Several changes can be made to the embodiments described in detail, all anyhow remaining within the protection scope of the invention, defined by the following claims.

## Claims

1. Vapor diffuser (1) comprising a handle (2) adapted to be gripped by the hand of a user; said handle (2) comprising an inlet (3) for the vapor coming from a boiler, a heat exchanger (4) accommodated inside said handle (2), and an outlet for delivering high temperature vapor, **characterized by** comprising a delivering device adapted to be mounted at the outlet (5) comprising a bushing (6) and a grid (8) comprising a plurality of through-holes (7) for the passage of the vapor; said grid (8) and said bushing (6) being made of conductive material.

2. Vapor diffuser (1) according to claim 1, **characterized in that** said bushing (6) extends around an axis (X-X) and has a substantially cylindrical shape for at least 50% of its extent; said bushing (6) has a first end placed at said outlet (5) and a second end away from said outlet (5).

3. Vapor diffuser (1) according to claim 2, **characterized in that** said grid (8) extends transversely to said axis (X-X) at a distance of at least 10 mm from the outlet (5).

4. Vapor diffuser (1) according to claim 2 or 3, **characterized in that** said grid (8) extends transversely to said axis (X-X) at a maximum distance of 40 mm from the outlet (5).

5. Vapor diffuser (1) according to any one of claims 1 to 4, **characterized in that** said grid (8) comprises a surface of at least 18 mm².

6. Vapor diffuser (1) according to any one of claims 1 to 4, **characterized in that** said grid (8) comprises a surface of up to 1500 mm².

7. Vapor diffuser (1) according to claim 1, **characterized in that** the holes (7) of said grid have a maximum size greater than or equal to 0.2 mm.

8. Vapor diffuser (1) according to claim 1, **characterized in that** the holes (7) of said grid have a maximum size less than or equal to 3 mm.

9. Vapor diffuser (1) according to claim 1, **characterized in that** said bushing (6) and said grid are made of metal alloys.
